Europäisches Patentamt

European Patent Office

Office européen des brevets

(11) Publication number: **0 303 088 B1**

# EUROPEAN PATENT SPECIFICATION

(45) Date of publication of patent specification: **11.11.92**

(51) Int. Cl.⁵: **A61K 39/395**, A61K 39/40, C07K 3/28, //C12P21/00

(21) Application number: **88111931.7**

(22) Date of filing: **25.07.88**

(54) **Purified IgM.**

(30) Priority: **10.08.87 US 83136**

(43) Date of publication of application:
**15.02.89 Bulletin 89/07**

(45) Publication of the grant of the patent:
**11.11.92 Bulletin 92/46**

(84) Designated Contracting States:
**AT BE CH DE ES FR GB GR IT LI LU NL SE**

(56) References cited:
**EP-A- 0 162 462**
**EP-A- 0 163 493**
**EP-A- 0 177 836**

**CHEMICAL ABSTRACTS, vol. 107, no. 5, 03 August 1987, Columbus, OH (US); F.STEINDL et al., p. 513, no. 37654v**

**PATENT ABSTRACTS OF JAPAN, vol. 6, no. 85 (C-103)[963], 22 May 1982**

**CHEMICAL ABSTRACTS, vol. 105, no. 19, 10 November 1986, Columbus, OH (US); F.CORMONT et al., p. 549, no. 170135b**

(73) Proprietor: **MILES INC.**
**1127 Myrtle Street**
**Elkhart Indiana 46514(US)**

(72) Inventor: **Dove, George**
**154 Goldenrod**
**Hercules, CA 94547(US)**
Inventor: **Mitra, Gautam**
**40 Cowper Avenue**
**Kensington, CA 94707(US)**

(74) Representative: **Adrian, Albert, Dr. et al**
**c/o BAYER AG Konzernverwaltung RP Patentabteilung**
**W-5090 Leverkusen 1, Bayerwerk(DE)**

CHEMICAL ABSTRACTS, vol. 106, no. 15, 13 April 1987, Columbus, OH (US); E.JOHNSON et al., p. p. 460, no. 117691z

JOURNAL OF IMMUNOLOGICAL METHODS, vol. 91, no. 2, June 1986, Elsevier Science Publishers B.V.; S.H.NEOH et al., pp. 231-235

PATENT ABSTRACTS OF JAPAN, vol. 9, no. 41 (C-267)[1764], 21 February 1985

CHEMICAL ABSTRACTS, vol. 109, no. 23, 05 December 1988, Columbus, OH (US); D.R.NAU, p. 502, nos. 209257c, 209254z

CHEMICAL ABSTRACTS, vol. 109, no. 7, 15 August 1988, Columbus, OH (US); F.M.CHEN et al., p. 505, no. 52793z

## Description

This disclosure is concerned generally with purified immunoglobulins and specifically with a highly purified immunoglobulin of the IgM class which is substantially free of nucleic acids.

IgM is a well known 19S immunoglobulin which comprises about 7% of the immunoglobulins found in man. IgM antibodies are said to have an antibody valence of at least five and they are the earliest antibodies generated in an immune response. Although IgM antibodies tend to be very effective, especially in combating bacterial infections, they have a relatively short in vivo half life of about five days. Further, IgM antibodies are labile and relatively difficult to stabilize, especially in purified form.

Various purification schemes have been suggested for plasma-derived IgM and, more recently, monoclonal-derived IgM. In the case of plasma-derived IgM, it has been known since the 1940's that alcohol fractionation techniques could be used to obtain a relatively concentrated IgM from what is known as Cohn Fraction III. See for example U.S. Patent 4,318,902 (and the cited references) to W. Stephan and concerned with the use of beta-propriolactone to make a concentrated IgM suitable for intravenous (IV) administration. In addition, see EPO application 0 038 667 of Miura et al (IgM acylation). See also, U.S. Patent No. 4,272,521 to Zuffi concerned with the purification of immune serum globulins in general by using ion exchange resins at an alkaline pH. Other IgM purification or preparation techniques are disclosed by U. Sugg et al, Vox Sang. 36:25 - 28 (1979); M. Steinbach et al, Preparative Biochemistry 3(4), 363 - 373 (1973) and A. Wichman et al, Biochem. Biophys. Acta 490:363 - 69 (1977). Techniques for making specific monoclonal antibodies of the IgM type are shown in U.S. Patent 4,271,145 to Wands et al. A specific immunoassay using high affinity IgM antibodies is disclosed in W 082/01072 published in the names of Wands et al. See also, I. A. Sampson et al, J. Immuno. Meth. 69, pp. 9 - 15, 1984. For a variety of technical reasons, plasma derived IgM has been relatively difficult to purify and the highest known purity to date is about 90% IgM, by weight. Also, the nucleic acid content of such plasma derived IgM has generally not been a serious concern because the IgM is derived from a human plasma source.

Typical nucleic acid contents for plasma-derived IgM are thought to be in the range of about 1 ng to 10 $\mu$g per mg.

Since the publication by Köhler and Milstein, "Continuous Cultures of Fused Cells Secreting Antibody of Predefined Specificity", Nature 256:495 - 497 (1975), the production of monoclonal antibodies has become well known. Monoclonal antibodies of a given specificity are now routinely made using somatic cell hybrids (see, for example, U.S. Patent No. 4,172,124 to H. Koprowski et al), using EBV transformed cells (see, for example, U.S. Patent No. 4,446,465 to M. Lostrom), a combination of the two or by the electrofusion of cells. Monoclonals of both the IgG and IgM classes have been made, purified and characterized. Such IgM preparations are described by D. Nau, Biochromatography, 1, No. 2, pp. 83 - 84 (95% pure IgM from tissue culture); M. Fishner, U.S. Patent No. 4,604,235 (90% pure IgM from mouse asciter fluid and which was characterized as "essentially pure antibody"); J. R. Wands et al, W 082/01072 (high affinity IgM monoclonal antibodies for diagnostics, cited above); S. Burchiel, et al, J. Immuno. Meth., 69, p. 33, 1984 (IgG purified from mouse asciter fluid); J. Deschamps et al, Anal. Biochem. 147, p. 451, 1985 (IgG from mouse asciter fluid); and T. Brooks et al, Amer. Lab., October, 1985 (use of hydroxyapatite for purification of mouse and human IgG and IgM). Although efforts have been made to purify IgM obtained from monoclonal sources, the highest reported IgM purity to date is about 95% (see Nau, above).

The preparation of a monoclonal IgM against P. aeruginosa has been disclosed and IgM derived from a human lymphoblastoid tissue culture and DEAE Sephacel™ has been used for an initial purification of the IgM. Therapeutically acceptable isotonic solutions of IgM with a concentration of 0.005 to 0.5 ug/ml are known but no data has been given on the relative purity of the IgM product or its formulation.

Although nucleic acid content of plasma-derived IgM has not aroused significant concern, the nucleic acid content of monoclonal IgM is very significant because of the potential danger of introducing foreign (non-human) nucleic acid into a human via a parenterally administered product. Hence, in addition to the desirability of obtaining a purified and concentrated IgM product, it is also desirable to obtain such a product with no or very little nucleic acid. We have now found that such a purified product can be prepared and stabilized by carefully controlling the processing steps and storage conditions. Details of our highly purified IgM are described below.

Our disclosure is concerned with an essentially pure and stabilized IgM antibody product comprising IgM antibodies having a purity greater than about 98% by weight and a nucleic acid content of less than about 200 pg per mg IgM. In preferred embodiments, the IgM purity is greater than 98% by weight, the nucleic acid content is less than 10 pg per mg IgM, most preferably the IgM purity is greater than about 99 % by weight, the nucleic

3

acid content is less than about 4 pg per mg IgM, and the preparation is stabilized by maintaining at a pH ranging from about 4 to 10, preferably at a pH of about 8 in the presence of NaCl, albumin, amino acids, or carbohydrates as stabilizers. An illustrative preparation having the above characteritics comprises one or more IgM antibodies specific to serotypic determinants found on the surface of gram negative microorganisms. In a preferred embodiment the preparation comprises IgM antibodies obtained from one or more clones and it is contemplated that it will be found useful in treating infections of P. aeruginosa. The preparation may be obtained by culturing a monoclonal antibody source, harvesting the monoclonal antibodies and then subjecting the harvested antibodies to a carefully controlled series of processing steps which include ion exchange and size exclusion chromatography.

The Figure is a flow chart showing the general process steps used to prepare one embodiment of this disclosure. Also shown are the successive increases in purity and decreases in nucleic acid content resulting from individual steps of the overall process.

A very important aspect of this disclosure is overall purity, stability and low nucleic acid content of our IgM preparation. As used herein, the expression "essentially pure and stabilized IgM" refers to an IgM preparation comprising IgM antibodies having a purity greater than about 98% by weight and a nucleic acid content of less than about 200 pg per mg of IgM. A "stabilized IgM preparation" means a preparation for which there is less than a 10% change (+ or -) in molecular weight distribution as measured by size exclusion chromatography over a period of at least 6 months (e.g., Pharmacia FPLC - Superose™ 6 peak area). The IgM antibodies are biologically active (capable of forming immunocomplexes) and stabilized by maintaining them at a pH ranging from about 4 to 10 in the presence of suitable stabilizing agents such as NaCl, albumin carbohydrates or amino acids. Examples for a carbohydrate stabilizer might be dextrose, sucrose or maltose. Although the reduced nucleic acid content is desirable in obtaining near homogeneity of the IgM preparation, regardless of IgM source, it is especially desirable in any IgM product obtained from cultures (e.g. of hybridoma or transformed cells) because of the importance of assuring the absence or near absence of nucleic acids (DNA or RNA) from a foreign source (animal origin or even human cells that have been genetically altered as, for example, by EBV transformation).

The illustrative examples below show an essentially pure and stabilized IgM specific to certain serotypes of Pseudomonas aeruginosa bacteria.

The IgM antibodies which we were able to purify and stabilize were generated from the following A.T.C.C. clones: line 6F11, Fisher Type 2, A.T.C.C. Accession No. CRL 8562, line 5G2, Fisher Type 6, A.T.C.C. Accession No. CRL 8797, and line 13C1, Fisher Type 5, A.T.C.C. Accession No. CRL 8796.

The following examples illustrate that monoclonal antibodies of class M and various Fisher types may be purified to high degree from tissue culture broths.

Example 1

Cell line 6F11, A.T.C.C. Accession No. CRL 8562 is a human lymphoblastoid cell producing monoclonal antibodies of class M specific to Fisher type 2 Pseudomonas aeruginosa. The line was grown in a mixture of Hana Biologics complex media supplemented with human serum albumin, insulin, and transferrin. The fermenter was a stirred tank.

A volume of 40 liters at 50 mg/l IgM obtained from the above culture was filtered through a 0.2 um filter (Microgon™) The filtrate was concentrated on a tangential flow membrane of 100,000 molecular weight cut-off (Millipore™) o 1 liter. The concentrate was cooled to 5° C and adjusted to pH 7.4. 100 g PEG was added and stirred for 1 hour. The solution was centrifuged at 10,000 x g for 30 minutes. The supernatant was discarded and the precipitate was frozen at -35° C.

The precipitate was resuspended in 1 liter of buffer (0.05 M TRIS, 0.08 M NaCl, pH 8.0). The pH was lowered to 4.5. The solution was centrifuged at 10,000 x g for 30 minutes and the precipitate was discarded. The supernatant was readjusted to pH 8.0. The solution was bound to an anion exchange column of 1 liter DEAE-Sepharose™ Fast-Flow (Pharmacia) equilibrated with buffer (0.05 M TRIS, 0.08 M NaCl, 2% TWEEN™ pH 8.0). The IgM was eluted by linear gradient with buffer (0.05 M TRIS, 1.0 M NaCl, pH 8.0). The eluate was concentrated on 100,000 m.w. membrane to 0.5 liters. The concentrate was fractionated on a size exclusion column of Sepharose™ CL-6B (Pharmacia) equilibrated with buffer (0.5 M NaCl, 0.05 M Tris, 0.01 M glycine, pH 8.0. The IgM eluate was 6 liters.

0.5 g human serum albumin was added and the pool was concentrated on 10,000 mw.w. membrane to 0.1 liters. The solution was diafiltered with 0.5 liters of buffer (0.15 M NaCl, 0.05 M TRIS, 0.01 M glycine, pH 8.0). The solution was sterile filtered. A sublot was frozen and lyophilized by a 80 hour cycle (10 hours at -40° C, 20 hours at -20°, 20 hours at -0°, 10 hours at 20°, and 20 hours at 37° C).

Cumulative yield is 30 - 35%. The liquid remains clear without precipitation for more than a year at 5° C. The lyophilized product is white and reconstitutes within 3 minutes with water. Purity is greater than 98% by SDS-PAGE and Pharmacia FPLC-Sepharose™ 6. Nucleic acid content by hybridization probe assay is less than 67 picogram/mg IgM.

Example 2

Cell line 5G2, A.T.C.C. Accession No. CRL 8797, is a human lymphoblastoid cell producing monoclonal antibodies of class M specific to Fisher type 6 of Pseudomonas aeruginosa. The line was grown by techniques essentially identical to the line in Example 1.

The broth was purified to final product by techniques similar to the line in Example 1, except that the initial 0.2 um filtrate was adjusted to pH 4.0 and held for 2 hours. The solution was readjusted to neutral pH and further steps were resumed (e.g., concentration, etc.). However, the volume of broth was 10 liters at 80 mg/l and other volumes were scaled proportionately. Final formulation buffer was 0.15 M NaCl, 0.01 M glycine, pH 8.0.

Cumulative yield is 30 - 35%. The liquid remains clear without precipitation for more than 6 months at 5° C. The lyophilized product is white and reconstitutes within 3 minutes with water. Purity is greater than 98% by SDS-PAGE and Pharmacia FPLC-Sepharose™ 6. Nucleic acid content by hybridization probe assay is less than 8.5 picogram/mg IgM.

Example 3

Cell line 13Cl, A.T.C.C. No. 8796, is a human lymphoblastoid cell producing monoclonal antibodies of class M specific to Fisher type 5 Pseudomonas aeruginosa. The line was grown by techniques essentially identical to the line in Example 1.

The broth was purified to final product by techniques essentially identical to the line in Example 2. However, the volume of broth was 10 liters at 100 mg/l and other volumes were scaled proportionately. Final formulation buffer was 0.15 M NaCl, 0.01 M glycine, pH 8.0

Cumulative yield is 30 - 35%. The liquid remains clear without precipitation for more than 6 months at 5° C. The lyophilized product is white and reconstitutes within 3 minutes with water. Purity is greater than 98% by SDS-PAGE and Pharmacia FPLC-Sepharose™ 6. Nucleic acid content by hybridization probe assay is less than 4 picogram/mg IgM.

Our general process is outlined in the Figure.

It was found that the highly purified product could be stabilized by adjusting it to a concentration ranging from 0.01 mg ml to 50 mg ml and a pH ranging from 4 to 10, preferably in the presence of NaCl, albumin, amino acids or carbohydrates, most preferably in the presence of about 0 to 5 wt. percent human serum albumin, about 0 to 10 % maltose, about 0.0 to 0.5 M NaCl and about 0 to 0.01 M glycine. Final product may be liquid (as above) or lyophilized and subjected to known techniques for inactivation of infectious agents.

Claims

Claims for the following Contracting States :
AT, BE, CH, DE, FR, GB, IT, LI, LU, NL, SE

1. A highly purified antibody preparation comprising antibodies of the IgM type having a purity greater than about 98 % by weight and a nucleic acid content of less than about 200 pg per mg IgM.

2. The preparation of claim 1 wherein the amount of nucleic acids in less than about 10 pg per mg IgM.

3. The preparation of claim 1 wherein the amount of nucleic acids is less than about 4 pg per mg IgM.

4. The preparation of claim 1 wherein the IgM antibodies are specific to antigens found on pathogenic gram negative microorganisms.

5. A highly purified monoclonal antibody preparation comprising monoclonal antibodies of the IgM type having a purity greater than about 98 % by weight and a nucleic acid content of less than about 200 pg per mg IgM.

6. The preparation of claim 5 having a nucleic acid content of less than about 10 pg per mg IgM.

7. The preparation of claim 5 having a nucleic acid content of less than about 4 pg per mg IgM.

8. The preparation of claim 5 having an IgM purity greater than about 99 % by weight.

9. The preparation of claim 5 wherein the antibody is specific to antigens of a gram negative microorganism.

10. The preparation of claim 5 wherein the antibody is specific to antigens of Pseudomonas aeruginosa bacteria.

11. The preparation of claim 5 including stabilizing amounts of human serum albumin.

**12.** The preparation of claim 5 including stabilizing amounts of a carbohydrate.

**13.** The preparation of claim 5 in an aqueous form having a pH ranging from about 4 to about 10.

**14.** The preparation of claim 5 wherein the carbohydrate is selected from dextrose, sucrose, and maltose.

**15.** The preparation of claim 5 in an aqueous form having a pH ranging from about 4 to about 10,0 to 5 wt. percent human serum albumin, about 0 to 10 % maltose, about 0.0 to 0.5 M NaCl and about 0 to 0.01 M glycine.

**Claims for the following Contracting States : ES, GR**

**1.** A method of preparation of a highly purified antibody preparation comprising antibodies of the IgM type having a purity greater than about 98 % by weight and a nucleic acid content of less than about 200 pg per mg IgM, characterized in that the preparation is conducted at a pH ranging from about 4 to 10 in the presence of NaCl and albumin as stabilizers.

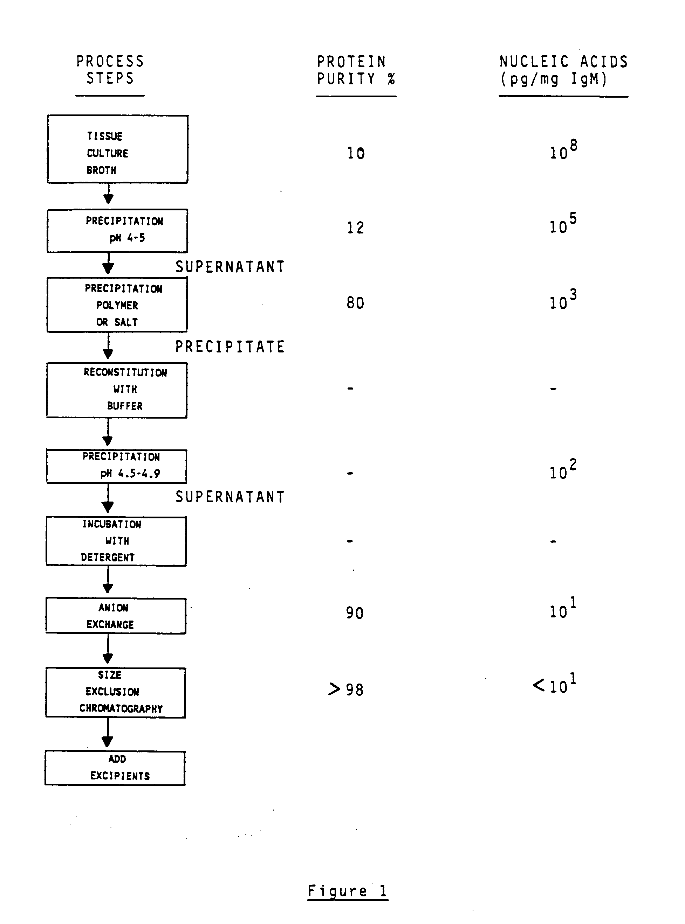

**2.** The method according to claim 1 wherein the preparation is subjected to the order of steps disclosed in figure 1.

**3.** The method according to claim 1 or 2 wherein the antibody is a monoclonal antibody of the IgM type.

**4.** The method according to claims 1 to 3 wherein in said preparation the amount of nucleic acids is less than about 10 pg per mg IgM.

**5.** The method according to claims 1 to 4 wherein in said preparation the amount of nucleic acids is less than about 4 pg per mg IgM

**6.** The method according to claims 1 to 5 wherein in said preparation the IgM antibodies are specific to antigens found on pathogenic gram negative microorganisms.

**7.** The method of the claims 1 to 6 wherein in said preparation the IgM purity is greater than about 99 % by weight.

**8.** The method of claims 1 to 7 wherein in said preparation the antibody is specific to antigens of Pseudomonas aeruginosa bacteria.

**9.** The method according to claims 1 to 8 wherein said preparation includes stabilizing amounts of a carbohydrate.

**10.** The method of claims 1 to 10 wherein said preparation is in aqueous form having a pH ranging from about 4 to about 10, 0 to 5 wt. percent human serum albumin, about 0 to 10 % maltose, about 0.0 to 0.5 M NaCl and about 0 to 0.01 M glycine.

**Patentansprüche**
**Patentansprüche für folgende Vertragsstaaten : AT, BE, CH, DE, FR, GB, IT, LI, LU, NL, SE**

**1.** Hochgereinigte Antikörperpräparation, enthaltend Antikörper vom IgM-Typ mit einer Reinheit größer als 98 Gew.-% und einem Nukleinsäuregehalt von weniger als 200 pg/mg IgM.

**2.** Präparation nach Anspruch 1, worin die Menge an Nukleinsäuren weniger als ca. 10 pg/mg IgM ist.

**3.** Präparation nach Anspruch 1, worin die Menge an Nukleinsäuren weniger als ca. 4 pg/ml IgM ist.

**4.** Präparation nach Anspruch 1, worin die IgM-Antikörper spezifisch gegen Antigene auf pathogenen gramnegativen Mikroorganismen gerichtet sind.

**5.** Hochgereinigte monoklonale Antikörperpräparation, enthaltend monoklonale Antikörper vom IgM-Typ mit eienr Reinheit größer als ca. 98 Gew.-% und einem Nukleinsäuregehalt von weniger als ca. 200 pg/mg IgM.

**6.** Präparation nach Anspruch 5 mit einem Nukleinsäuregehalt von weniger als ca. 10 pg/mg IgM.

**7.** Präparation nach Anspruch 5 mit einem Nukleinsäuregehalt von weniger als ca. 4 pg/mg IgM.

**8.** Präparation nach Anspruch 5 mit einer IgM-Reinheit größer als ca. 99 Gew.-%.

**9.** Präparation nach Anspruch 5, worin der Antikörper spezifisch gegen Antigene von gramnegativen Mikroorganismen gerichtet ist.

**10.** Präparation nach Anspruch 5, worin der Antikörper spezifisch gegen Antigene vom Pseudomonas aeruginosa-Bakterien gerichtet ist.

**11.** Präparation nach Anspruch 5, die stabilisierende Mengen von humanem Serumalbumin enthält.

**12.** Präparation nach Anspruch 5, die stabilisierende Mengen eines Kohlenhydrats enthält.

**13.** Präparatiion nach Anspruch 5 in einer wäßrigen Form mit einem pH im Bereich von ca. 4 bis ca. 10.

**14.** Präparation nach Anspruch 5, worin das Kohlenhydrat ausgewählt ist aus Dextrose, Saccharose und Maltose.

**15.** Präparation nach Anspruch 5 in wäßriger Form mit einem pH im Bereich von ca. 4 bis ca. 10, 0 bis 5 Gew.-% menschlichem Serumalbumin, ca. 0 bis 10 % Maltose, ca. 0,0 bis 0,5 M NaCl und ca. 0 bis 0,01 M Glycin.

**Patentansprüche für folgende Vertragsstaaten : ES, GR**

**1.** Verfahren zur Herstellung einer hochgereinigten Antikörperpräparation, die Antikörper des IgM-Typs mit einer Reinheit von größer 98 Gew.-% und einem Nukleinsäuregehalt von weniger als ca. 200 pg/mg IgM enthält, dadurch gekennzeichnet, daß die Herstellung bei einem pH-Bereich von ca. 4 bis 10 in Gegenwart von NaCl und Albumin als Stabilisatoren durchgeführt wird.

**2.** Verfahren nach Anspruch 1, worin die Präparation der Reihe nach der in Fig. 1 gezeigten Schritte unterworfen wird.

**3.** Verfahren nach Anspruch 1 oder 2, worin der Antikörper ein monoklonaler Antikörper vom IgM-Typ ist.

**4.** Verfahren nach Ansprüchen 1 bis 3, worin in der Präparation die Menge der Nukleinsäuren weniger als 10 pg/mg IgM ist.

**5.** Verfahren nach den Ansprüchen 1 bis 4, worin in der Präparation die Menge der Nukleinsäuren weniger als ca. 4 pg/mg IgM ist.

**6.** Verfahren nach den Ansprüchen 1 bis 5, worin in der Präparation die IgM-Antikörper spezifisch gegen Antigene auf pathogenen gramnegativen Mikroorganismen gerichtet sind.

**7.** Verfahren nach den Ansprüchen 1 bis 6, worin in der Präparation die IgM-Reinheit größer als ca. 99 Gew.-% ist.

**8.** Verfahren nach den Ansprüchen 1 bis 7, worin in der Präparation der Antikörper spezifisch gegen Antigene vom Pseudomonas aeruginosa-Bakterien gerichtet ist.

**9.** Verfahren nach den Ansprüchen 1 bis 8, worin die Präparation stabilisierende Mengen eines Kohlenhydrats enthält.

**10.** Verfahren nach den Ansprüchen 1 bis 9, worin die Präparation in einer wäßrigen Form vorliegt mit einem pH im Bereich von ca. 4 bis ca. 10, 0 bis 5 Gew.-% menschlichem Serumalbumin, ca. 0 bis 10 % Maltose, ca. 0,0 bis 0,5 M NaCl und ca. 0 bis 0,01 M Glycin.

**Revendications**
**Revendications pour les Etats contractants suivants: AT, BE, CH, DE, FR, GB, IT, LI, LU, NL, SE**

**1.** Préparation d'anticorps hautement purifiés, comprenant des anticorps du type IgM ayant une pureté supérieure à environ 98 % en poids et une teneur en acides nucléiques inférieure à environ 200 pg par mg d'IgM.

**2.** Préparation suivant la revendication 1, dans laquelle la quantité d'acides nucléiques est inférieure à environ 10 pg par mg d'IgM.

**3.** Préparation suivant la revendication 1, dans laquelle la quantité d'acides nucléiques est inférieure à environ 4 pg par mg d'IgM.

**4.** Préparation suivant la revendication 1, dans laquelle les anticorps IgM sont spécifiques d'antigènes présents sur des micro-organismes pathogènes Gram-négatifs.

**5.** Préparation d'anticorps monoclonaux hautement purifiés, comprenant des anticorps monoclonaux du type IgM ayant une pureté supérieure à environ 98 % en poids et une teneur en acides nucléiques inférieure à environ 200 pg par mg d'IgM.

**6.** Préparation suivant la revendication 5, ayant une teneur en acides nucléiques inférieure à environ 10 pg par mg d'IgM.

**7.** Préparation suivant la revendication 5, ayant une teneur en acides nucléiques inférieure à environ 4 pg par mg d'IgM.

**8.** Préparation suivant la revendication 5, ayant une pureté des IgM supérieure à environ 99 % en poids.

**9.** Préparation suivant la revendication 5, dans laquelle l'anticorps est spécifique d'antigènes d'un microorganisme Gram-négatif.

**10.** Préparation suivant la revendication 5, dans laquelle l'anticorps est spécifique d'antigènes de bactéries de l'espèce Pseudomonas aeruginosa.

**11.** Préparation suivant la revendication 5, comprenant des quantités stabilisantes de sérum-albumine humaine.

**12.** Préparation suivant la revendication 5, comprenant des quantités stabilisantes d'un glucide.

**13.** Préparation suivant la revendication 5, sous une forme aqueuse ayant un pH compris dans l'intervalle d'environ 4 à environ 10.

**14.** Préparation suivant la revendication 5, dans laquelle le glucide est choisi entre le dextrose, le saccharose et le maltose.

**15.** Préparation suivant la revendication 5, sous une forme aqueuse ayant un pH compris dans l'intervalle d'environ 4 à environ 10 et comprenant 0 à 5 % en poids de sérum-albumine humaine, environ 0 à 10 % de maltose, du NaCl approximativement 0,0 à 0,5 M et de la glycine approximativement 0 à 0,01 M.

**Revendications pour les Etats contractants suivants : ES, GR**

**1.** Procédé de production d'une préparation d'anticorps hautement purifiés comprenant des anticorps du type IgM ayant une pureté supérieure à environ 98 % en poids et une teneur en acides nucléiques inférieure à environ 200 pg par mg d'IgM, caractérisé en ce qu'il est mis en oeuvre à un pH d'environ 4 à 10 en présence de NaCl et d'albumine servant de stabilisants.

**2.** Procédé suivant la revendication 1, dans lequel la préparation est soumise à une série d'étapes dans l'ordre représenté sur la figure 1.

**3.** Procédé suivant la revendication 1 ou 2, dans lequel l'anticorps est un anticorps monoclonal du type IgM.

**4.** Procédé suivant les revendications 1 à 3, dans lequel, dans la préparation, la quantité d'acides nucléiques est inférieure à environ 10 pg par mg d'IgM.

**5.** Procédé suivant les revendications 1 à 4, dans lequel, dans la préparation, la quantité d'acides nucléiques est inférieure à environ 4 pg par mg d'IgM.

**6.** Procédé suivant les revendications 1 à 5, dans lequel, dans la préparation, les anticorps IgM sont spécifiques d'antigènes présents sur des micro-organismes pathogènes Gram-négatifs.

**7.** Procédé suivant les revendications 1 à 6, dans lequel, dans la préparation, la pureté des IgM est supérieure à environ 99 % en poids.

**8.** Procédé suivant les revendications 1 à 7, dans lequel, dans la préparation, l'anticorps est spécifique d'antigènes de bactéries de l'espèce Pseudomonas aeruginosa.

**9.** Procédé suivant les revendications 1 à 8, dans lequel la préparation comprend des quantités stabilisantes d'un glucide.

**10.** Procédé suivant les revendications 1 à 9, dans lequel la préparation est sous une forme aqueuse ayant un pH compris dans l'intervalle d'environ 4 à environ 10 et comprend 0 à 5 % en poids de sérum-albumine humaine, environ 0 à 10 % de maltose, du NaCl approximativement 0,0 à 0,5 M et de la glycine approximativement 0 à 0,01 M.

| PROCESS STEPS | PROTEIN PURITY % | NUCLEIC ACIDS (pg/mg IgM) |
|---|---|---|
| TISSUE CULTURE BROTH | 10 | $10^8$ |
| PRECIPITATION pH 4-5 | 12 | $10^5$ |
| SUPERNATANT | | |
| PRECIPITATION POLYMER OR SALT | 80 | $10^3$ |
| PRECIPITATE | | |
| RECONSTITUTION WITH BUFFER | - | - |
| PRECIPITATION pH 4.5-4.9 | - | $10^2$ |
| SUPERNATANT | | |
| INCUBATION WITH DETERGENT | - | - |
| ANION EXCHANGE | 90 | $10^1$ |
| SIZE EXCLUSION CHROMATOGRAPHY | > 98 | $< 10^1$ |
| ADD EXCIPIENTS | | |

Figure 1

9